**Europäisches Patentamt**

**European Patent Office**

**Office européen des brevets**

(19)

(11) Publication number: **0 134 247**

**A1**

(12) # EUROPEAN PATENT APPLICATION
published in accordance with Art 158(3) EPC

(21) Application number: **84900640.8**

(22) Date of filing: **26.01.84**

Data of the international application taken as a basis:

(86) International application number:
**PCT/JP84/00019**

(87) International publication number:
**WO84/02912 (02.08.84 84/18)**

(51) Int. Cl.4: **C 07 G 7/00**
**C 12 P 21/00, A 61 K 37/02**
**//(C12P21/00, C12R1/91)**

(30) Priority: **31.01.83 JP 15290/83**

(43) Date of publication of application:
**20.03.85 Bulletin 85/12**

(84) Designated Contracting States:
**CH DE FR GB LI NL SE**

(71) Applicant: **OTSUKA PHARMACEUTICAL CO., LTD.**
**9, Kandatsukasacho 2-chome**
**Chiyoda-ku Tokyo 101(JP)**

(72) Inventor: **ITAYA, Kimikazu 1-18, Aza-niwake**
**Shinkirai, Kitajimacho**
**Itano-gun Tokushima 772-02(JP)**

(72) Inventor: **ISHI, Kiyoshi 39-46, Aza-sakafuji**
**Sumiyoshi, Aizumicho Itano-gun**
**Tokushima 771-12(JP)**

(72) Inventor: **MIZUTA, Kazutaka 52-11,**
**Aza-minamikawamukai**
**Hiroshima, Matsushigecho**
**Itano-gun Tokushima 771-02(JP)**

(72) Inventor: **KANETA, Hideo 415-12, Kagasuno**
**Kawauchicho, Tokushima-shi**
**Tokushima 771-01(JP)**

(72) Inventor: **SHIGENAGA, Toshiaki 127-2, Kawakubo**
**Tainohama, Kitajimacho**
**Itano-gun Tokushima 771-02(JP)**

(72) Inventor: **KUJIRA, Kenichi 35-17,**
**Aza-minamikawamukai**
**Hiroshima, Matsushigecho**
**Itano-gun Tokushima 771-02(JP)**

(72) Inventor: **YAMANISHI, Kazuya 463-10, Kagasuno**
**Kawauchicho, Tokushima-shi**
**Tokushima 771-01(JP)**

(74) Representative: **Kressin, Horst-Rüdiger, Dr.**
**Herzog-Wilhelm-Strasse 16**
**D-8000 München 2(DE)**

(54) **PROTEIN WITH ONCOSTATIC EFFECT, PROCESS FOR ITS PREPARATION, AND ONCOSTATIC DRUG CONTAINING IT.**

(57) Protein having he following properties: a)M.W.: 56,000 − 3,000; b) isoelectric point: PH 4.9 − 0.3 c) showing a cytotoxic effect on cultured mouse L-cells; and d) showing an oncostatic effect on female A sarcoma tumor-carrying mouse. and a process for recovering the protein from an enriched medium of established lymphoid cells of human origin. The protein is produced by adding an inducer such as endotoxin. lectin or the like of virus or gram-negative bacterial origin to established lymphoid cells of human origin suspended in a enriched medium. and conducting culture The protein can be used as an oncostatic agent

EP 0 134 247 A1

SPECIFICATION  TITLE MODIFIED

see front page

TITLE OF THE INVENTION

PROTEIN HAVING ANTI-CANCER ACTIVITY, PROCESS FOR PRE-PARING SAME, AND ANTI-TUMOR COMPOSITIONS CONTAINING SAME

FIELD OF THE INVENTION

The present invention relates to a novel protein having anti-cancer activity, a process for preparing the protein, and anti-cancer compositions containing the same.

BACKGROUND ART

Carswell et al. found that when mice sensitized with bacillus Calmette Guerin (BCG) were given endotoxin on the 14th day, a factory having cytotoxicity to L-cells was produced in the serum in 2 hours and named the factor a tumor necrosis factor (TNF) (Proc. Nat. Acad. Sci. U.S.A., Vol. 72, p. 3666, 1975). Green et al. partially purified this substance by fractional precipitation with ammonium sulfate and gel filtration and reported that the TNF had a molecular weight of about 150000 (Proc. Nat. Acad. Sci., U.S.A., Vol. 73, p. 381, 1976). Matthews et al. later gave BCG to rabbits and administered endotoxin 2 weeks thereafter to produce TNF, which was then purified, and reported that the substance had a molecular weight of 39000 as determined by gel filtration method or 67000 by polyacrylamide gel electrophoresis (PAGE) (Br. J. Cancer,

0134247

- 2 -

Vol. 42, p. 416, 1980). Haranaka et al. further produced TNF with mice and rabbits using Propionibacterium acnes and endotoxin and reported that the substance had a molecular weight of 39000 as determined by gel filtration and PAGE (Nihon Rinsho, Vol. 40, p. 1872, 1982).

Thus, TNF has been expected to be useful for curing malignant tumors because of its physiological activity. However, because this substance is prepared from the serum of animals other than man, is difficult to produce on an industrial scale and to purify and is a protein component derived from animals other than man, it has problems as to antigenicity and also in respect of safety in treating patients. Attempts have been made to produce similarly physiologically active substances from human cultured cell lines. For example, Amino et al. reportedly produced a soluble factor (HLT-LCL) having cytotoxic activity on mouse L-cells by causing Phaseolus Vulgaris lectin (PHA) to act on human cultured lymphoid cell line M-7002 or B-7001 [The Journal of Immunology, Vol. 113, No.4, 1334-1345 (1974)].

According to the report, the soluble factor has a molecular weight of 68000 to 150000 as determined by gel filtration, but the substance per se has not been isolated and identified.

We have also made intensive research to identify the above substance having cytotoxicity on L-cells and consequently succeeded in isolating from a human cultured lymphoid cell line another purified protein having the above physiological activity but different from the reported soluble factor. We have found that this substance has anti-cancer activity and high safety in treating patients and accomplished the present invention.

DISCLOSURE OF THE INVENTION

The novel protein of the present invention is derived and produced by causing an anti-tumor substance inducing agent to act on human cultured lymphoid cell lines and is characterized by the following characteristics.

(1) Molecular weight

A. Molecular weight determined by gel filtration with use of Biogel A 1.5 m

The molecular weight of the protein was determined by packing Biogel A 1.5 m (product of Bio-Rad Lab., U.S.A.) into a column (16 x 1000 mm, product of Pharmacia Fine Chemicals , Sweden), using a buffer of 0.04 M tris-hydrochloric acid/0.1% sodium dodecylsulfate (SDS)/4M urea/0.1M NaCl (pH 7.8), adding thereto a sample of present substance in an amount of 200 µg (amount of protein) for gel filtration, and using a standard curve

- 4 -

which was determined by a molecular weight assay kit [product of Pharmacia Fine Chemicals, Sweden, markers: bovine serum albumin (molecular weight 67000), ovalbumin (molecular weight 43000), chymotrypsinogen A (molecular weight 25000) and ribonuclease (molecular weight 13700)] from the position of elution of the sample. The amount of protein was determined by pigment coupling with use of Coomassile Brilliant Blue G-250 according to the Bradford method (Anal. Biochem., Vol. 72, p. 248, 1976). The same method was used hereinafter.

Consequently the present substance had relative mobility at about 1.37, eluted before ovalbumin and had a molecular weight of about 55000.

B. Molecular weight determined by gel filtration with use of TSK Gel G3000SW

A sample of present substance (100 µg, in the amount of protein) was subjected to gel filtration with use of TSK Gel G3000SW (product of Toyo Soda Co., Ltd.) connected to Pharmacia FPLC system (product of Pharmacia Fine Chemicals, Sweden) and a buffer of 0.2 M NaCl/0.1M sodium phosphate (pH 7.0). The molecular weight of the sample was calculated from elution patterns with use of a molecular weight assay kit for high-speed liquid chromatography [product of Oriental Yeast Co., Ltd., markers: glutamic acid dehydrogenase (molecular weight 290000), enolase (molecular weight 67000), adenylate kinase (molecular weight 32000) and cytochrome C (molecular weight 12300)]. Consequently the present substance took about 40 minutes for elution, eluted before adenylate kinase and had a molecular weight of about 56000. .

C. Molecular weight determined by electrophoresis with use of SDS/polyacrylamide gel

The molecular weight of a sample of the present substance was determined according to the method of Kondo et al. (Seikagaku, Vol. 44, p. 304, 1972) by adding 5 μg (amount of protein) of the sample to SDS-polyacrylamide gel with sodium phosphate/SDS (pH 7.2), subjecting the sample to electrophoresis at 40 mA for 7 hours while recording electrophoretic patterns with use of a molecular weight assay kit (product of Pharmacia Fine Chemicals, Sweden, markers: the same as in above A.) and drawing a molecular weight curve from the patterns. Consequently the present substance had relative mobility at about 0.41 and a molecular weight of about 56000.

(2) Isoelectric point

The isoelectric point of the present substance was measured using an isoelectric point measuring device (product of Bio-Rad Lab., U.S.A.), Ampholine polyacrylamide plate (pH 3.5-9.5, product of LKB Biochrom Ltd.,

U.S.A.) and standard isoelectric point measuring marker kit (product of Pharmacia Fine Chemicals, Sweden). More specifically, a piece of filter paper was caused to absorb about 5 μg (amount of protein) of a sample of the present substance, the paper was placed on the gel, the sample was subjected to electrophoresis with constant power of 10 W for about 2 hours, and the electrophoresis was completed when the current became constant. The gel was cut into pieces at a spacing of 1 mm and subjected to elution with a buffer to measure the activity on L-cells. The isoelectric point was calculated with reference to the isoelectric point markers. Consequently the present substance was found to have an isoelectric point at pH of 4.9 ± 0.3.

(3) Solubility, color and properties

When a sample of the present substance is dissolved in 0.02 M tris-hydrochloric acid buffer (pH 7.8) to a concentration of 3 mg of protein/ml, the solution is colorless and transparent.

When acetone or ethanol is added to the solution to a concentration of at least 70 V/V %, precipitation occurs.

When the present substance is dissolved in water to a concentration of 3 mg of protein/ml, the solution is weakly acidic.

(4) Color reaction

The present substance is positive when tested for peptide bond and amino acid color reactions by burette reaction, Folin-Lowry reaction, and ninhydrin reaction after hydrolysis with hydrochloric acid.

The protein of the present invention is further characterized by the following physiological activities.

(a) Cytotoxic activity on L-cells

According to the method of above-mentioned Carswell and the method of Klostergaard (Mol. Imm., Vol. 17, p. 613, 1980), the present substance was checked for cytocidal effect on L-cells. L-cells were suspended at a conctration of $2 \times 10^5$ cells/ml in Eagle's minimal essential medium (MEM) containing 250 units/ml of penicillin and 125 μg/ml of streptomycin. The L-cell suspension (0.1 ml) and 0.1 ml of a sample of the present substance diluted to a suitable concentration were placed into each well of a microplate having 96 wells (product of Coster, U.S.A.). For incubation, the plate was held at 37°C for 48 hours in air containing 5% of carbon dioxide. The cultured cells were dyed with Neutral Red, and the number of live cells was colorimetrically determined by Titertek Multiscan (product of Flow Lab. Inc., U.S.A.). The activity is expressed in units multiplied by the number of times the sample was diluted, the effect to kill 50% of L-cells being one unit.

Consequently the cytotoxicity of the present substance on L-cells was found to be at least about $3.5 \times 10^6$ units/mg protein.

(b) Anti-tumor activity on mice with Meth A-sarcoma

$2 \times 10^5$ Meth A-sarcoma cells were transplanted intradermally at abdominal region of BALB/c mouse. Seven days thereafter when the tumor grew to a diameter of 7 to 8 mm, 0.2 ml of a sample of the present substance diluted to $5 \times 10^3$ to $5 \times 10^4$ units/ml in terms of cytotoxicity on L-cells as determined by the above method (a) was injected into the tail vein of the mouse. Forty-eight hours thereafter, the anti-tumor activity was checked by the method of Carswell et al. according to the following criteria.

(-)    : No change.

(+)    : Slight hemorrhagic necrosis.

(++)   : Medium degree of hemorrhagic necrosis (necrosis over at least 50% of the surface of the transplanted tumor at the center thereof).

(+++)  : Marked hemorrhagic necrosis (serious necrosis in the central portion of the transplanted tumor, with tumor tissues slightly left along its periphery).

Table 1 shows the results obtained.

Table 1

| Dose | Result | | | |
|---|---|---|---|---|
| (μg amount of protein/mouse) | - | + | ++ | +++ |
| - (Saline) | 6 | 0 | 0 | 0 |
| 0.286 | 1 | 3 | 2 | 0 |
| 0.572 | 0 | 3 | 2 | 1 |
| 1.43 | 0 | 1 | 4 | 1 |
| 2.86 | 0 | 1 | 2 | 3 |

The novel protein of the present invention is prepared by the following process.

The substance of the present invention is derived by causing an anti-tumor substance inducing agent to act on a human cultured lymphoid cell line. The cultured lymphoid cell line is not particularly limited. Usable cell lines include already established known lymphoid cell lines, and normal lymphoid cells which are treated with various viruses, chemicals, radiation, etc. and cultured into cell lines. Examples of such cell lines are T-cell, B-cell, myeloid cell, non-T-cell, non-B-cell and like cell lines which are disclosed, for example, in Immunol. Commun., 9(8), pp. 731-734 (1980), Tanpakushitsu Kakusan Koso, Vol. 23, No.6, pp. 291-305, and Seikagaku Data Book II, pp. 829-905, Kabushikikaisha Tokyo Kagaku Dojin, issued on June 23, 1980. Of these, especially suitable human cultured lymphoid cell lines are CCRF-CEM, DND-41, TALL-1, RPMI-8402 and CCRF-HSB-2 cells (all disclosed in Immunol. Commun., 9(8), pp. 731-734 (1980)).

Anti-tumor substance inducing agents which are to be caused to act on such cultured lymphoid cell lines are various known viruses, endotoxins derived from gram-negative bacteria, lectins derived from vegetables, substances having immunoactivity, etc. Typical examples of such agents are endotoxins derived from gram-negative bacteria, such as lipopolysaccharides (LPS) derived from Escherichia coli, Pseudomonas aeruginosa Salmonella typhimrium etc.; lectins derived from vegetables, such as Concanavalia Ensiformis lectin (concanavalin A, Con A), soybean lectin (SBA), Phaseolus Vulgaris lecin (PHA), etc.; viruses such as Sendai virus (HVJ); and bacillus Calmette Guerin (BCG), Corynebacterium parvum, Propionibacterium acnes, Mycobacterium butyricum, Corynebacterium granulosum, Streptococcus pyrogenes, Plasmodium, Zymosan, Nocardia asteroides, Lysteria monocytogenes, glucan, cellwall skelton, dextran sulfate, muramyldipeptide, Krestin (product of Kureha Kogyo Co., Ltd.) and other substances having immunoactivity. These agents can be used in combination.

When required, the cultured lymphoid cell line can be suitably incubated and proliferated before it is treated with the anti-tumor substance inducing agent. The incubation conditions are not limited particularly and can be those usually used. For example, a usual nutrient medium, such as RPMI-1640 or MEM medium, which is modified with a serum supplement such as fetal calf serum (FCS) is used for proliferation in vitro. Alternatively cells can be transplanted in a mammal other than man for proliferation in vivo. With the latter method, the animal used for transplant is likely to undergo an immune reaction with human cells, so that it is desirable to use infant animals, or nude mice or animals which are immunosuppressed by treatment with radiation at about 200 to 600 rem, anti-serum treatment, treatment with an immunosuppressive agent, etc. The human cultured cell line thus proliferated is suspended in the nutrient medium (usually having a pH of 6.5-8.0) at a concentration of about $1 \times 10^4$ to about $1 \times 10^7$ cells/ml, and the suspension is subjected to the action of the above-mentioned anti-tumor substance inducing agent, whereby the desired substance of the invention can be derived and produced. For this treatment, the inducing agent is used, for example at a concentration of about 1 to about 10 µg/ml in the case of endotoxin and immuno- active substances, about 10 to about 500 μg/ml for lectins, or about 50 to about 5000 HA/ml for viruses. The mixture is incubated usually at a temperature of 37°C for 1 to 6 days. Consequently the present substance is produced in the nutrient medium. (The liquid thus obtained will be hereinafter referred to as a "crude solution.")

The present substance is collected and isolated from the crude solution thus obtained and purified by a physicochemical or biochemical method making use of the properties of the substance contained in the crude solution. For example, salting-out, chromatography, electrophoresis, extraction, centrifugation, dialysis, etc. are resorted to in a suitable combination. Preferably the crude solution is subjected to the following steps.

(1)     Salting-out with ammonium sulfate

(2)     Gel filtration

(3)     Hydroxyapatite chromatography

(4)     Pharmacia FPLC mono Q column chromatography

(5)     Ammonium sulfate salting-out dissolving chromatography

(6)     Gel filtration

These steps will be described below in detail.

Purifying step 1

Ammonium sulfate is added to the crude solution to prepare a 50 to 80% saturated solution, which is then allowed to stand in a low-temperature chamber (4°C) for 2 hours to overnight to fully precipitate a physiologically active protein. Subsequently the solution is centrifuged at 10000 r.p.m. for 10 to 30 minutes with a cold centrifuge (product of Hitachi, Ltd.) to collect the physiologically active precipitate. This step achieves an activity collection ratio (as determined by the foregoing method of measuring cytotoxicity on L-cells) of 80 to 100% and a purifying degree of 10 to 20 times.

Purifying step 2

The precipitate obtained by step 1 is suspended in a buffer of 0.02M tris-hydrochloric acid (pH 7.8)/0.1M NaCl. To the suspension is added 2 to 8M urea, and the mixture is cold-centrifuged at 10000 r.p.m. for 10 to 30 minutes to separate off the insolubles and obtain a transparent physiologically active supernatant, which is then subjected to gel filtration. Useful gel filtration carriers are Ultragel AcA44, 54 (product of LKB Biochrom Ltd., U.S.A.), Biogel A 1.5 m (product of Bio-Rad Lab., U.S.A.), etc.

The fractions from gel filtration are checked for cytotoxicity on L-cells by the foregoing method, and the active fractions are collected and subjected to ultrafiltration for concentration by an ultrafiltration apparatus TCF-10 (product of Amicon Corp., U.S.A.) equipped with an ultrafilter YM10 (product of Amicon Corp., U.S.A.). The filtrate may be concentrated by precipitation with use of 50 to 80% saturated ammonium sulfate solution. This step achieves an activity collection ratio of 80 to 100% and a purifying degree of 20 to 50 times.

Purifying step 3

With use of a dialyzing tube (product of Nakarai Kagaku Yakuhin Co., Ltd.), the concentrate of physiologically active fractions obtained by step 2 is dialyzed overnight at 4°C with use of 0.02M phosphoric acid buffer (pH 6.8) in an amount of 50 to 100 times the amount of the concentrate while replacing the external liquid several times. The resulting dialyzate is centrifuged by a cold centrifuge (4°C) at 10000 r.p.m. for 20 to 30 minutes to obtain a transparent supernatant. Subsequently the supernatant is caused to be adsorbed by hydroxyapatite (product of Nippon Chemical Co., Ltd.) and subjected to elution with 0.02M to 0.5M phosphoric acid buffer (pH 6.8) by continuous concentration gradient method or by increasing the concentration stepwise to separate a physiologically active portion, which is then concentrated by ultrafiltration or salting-out with ammonium sulfate. This step achieves a physiologically active portion collecting ratio of 40 to 80% and a purifying degree of about 4 to 10 times.

Purifying step 4

The physiologically active concentrate resulting from purifying step 3 is placed into a dialyzing tube and dialyzed overnight at 4°C with a dilute phosphoric acid or tris buffer (pH 7.0 to 8.0) in an amount of 50 to 100 times the amount of the concentrate. The dialyzate is caused to be adsorbed by Pharmacia FPLC mono Q column (product of Pharmacia Fine Chemicals, Sweden), and a physiologically active substance is eluted by continuously increasing the concentration of a buffer or NaCl solution. The step achieves an activity collection ratio of 70 to 90% and a purifying degree of about 3 to about 10 times.

Purifying step 5

The active portion obtained by step 4 is concentrated by ultrafiltration and then subjected to ammonium sulfate salting-out and dissolving column chromatography. With use of a column packed with Toyo-pearl SW50, 55 or 60 (product of Toyo Soda Mfg. Co., Ltd.) and Pharmacia FPLC system (product of Pharmacia Fine Chemicals, Sweden), the active portion is subjected to salting-out with ammonium sulfate within the column and then dissolved for separation by continuously decreasing the concentration of ammonium sulfate.

This step achieves an activity collection ratio of 30 to 70% and a purifying degree of about 3 to about 10 times.

Purifying step 6

The active portion obtained by step 5 is concentrated and placed into a column (16 x 1000 mm) packed with Biogel A 1.5 m or Ultragel AcA44 or 54 for gel filtration. Alternatively Toyo Soda High-Speed Liquid Chromatographic Column G2000SW or G3000SW (product of Toyo Soda Mfg. Co., Ltd.) is used for gel filtration. This step achieves an activity collection ratio of 25 to 75% and a purifying degre of about 3 to about 15 times.

Purifying steps 1 to 6 achieve an overall activity collection ratio of 5 to 30% and an overall purifying degree of about $1.0 \times 10^5$ to about $1.0 \times 10^6$ times.

The physiologically active protein of the present invention thus obtained was checked for characteristics with the results as given above.

In this way, the protein of this invention is obtained. As already stated, the substance of the invention obtained has direct cytotoxic activity on L-cells in vitro and anti-tumor activity in vivo. As will be apparent from the following pharmacological test examples, the present substance further exhibits cytotoxic or cytocidal activity also on human cancer cells or melanoma cells and is low in toxicity.

Pharmacological Test Example I

Cytotoxic or cytocidal activity

(a) Cytocidal activity on human cancer cells

The present substance was tested for cytocidal effect on some cells, i.e. Raji, a cell line derived from human Burkitt's lymphoma (J. Nat. Cancer Inst., Vol. 37, p. 547, 1966), Kato-III, a cell line derived from human gastric cancer (signet ring cell cancer) (Jpn. J. Exp. Med., Vol. 48, p. 61, 1978) and KB, a cell line derived from human carcinoma of nasopharynx (Cancer Res., Vol. 18, p. 1017, 1958). Raji cells derived from human Burkitt's lymphoma and kato-III cells derived from human gastric cancer were suspended to a concentration of $2 \times 10^5$ cells/ml in RPMI 1640 medium containing 250 units/ml of penicillin, 125 µg/ml of streptomycin and 10% immobilized fetal calf serum. Further KB cells derived from human carcinoma of nasopharynx were suspended to a concentration of $2 \times 10^5$ cells/ml in Eagle's minimum essential medium containing 250 units/ml of penicillin, 125 µg/ml of streptomycin and 10% immobilized bovine serum.

A 0.1 ml quantity of each cell suspension and 0.1 ml of dilution of the present substance having a specified concentration were placed into each well of a microplate having 96 wells, and the plate was held at 37°C for 48 hours in air containing 5% carbon dioxide, whereby the cells were incubated.

After incubation for 48 hours, the cells were dyed with Trypan Blue, and the number of live cells was counted under a microscope with use of Burker-Turk hemacytometer (product of Erma Optical Works Co., Japan). The results revealed that the concentration of the present substance for inhibiting proliferation of cells 50% was at least 40 ng protein/ml for Raji cells, at least 140 ng protein/ml for kato-III cells or at least 56 ng protein/ml for KB cells.

(b) Cytotoxic activity on melanoma cells

The present substance was tested for cytotoxic activity on cells of melanoma A-375 (J. Natl. Cancer Inst., Vol. 51, p. 1417, 1973) according to the method of Helson et al. (Nature, Vol. 258, p. 731, 1975). A suspension containing melanoma A-375 cells in an amount of $5 \times 10^4$ cells/ml was prepared from Eagle's medium containing glutamine, non-essential amino acids, penicillin, streptomycin and 10% immobilized fetal calf serum. A 1 ml quantity of the cell suspension and 1 ml of a solution of present substance diluted to a suitable concentration were placed into a dish, 3.5 cm in diameter. The cells were incubated at 37°C in air containing 5% carbon dioxide.

On the third day of incubation, the cells were dyed with Trypan Blue, and the number of live cells was counted under a microscope with use of Burker-Turk hemacytometer as in the above test (a). It was found that the amount of the present substance required for inhibiting proliferation of melanoma A-375 cells 50% was at least 72 ng protein/ml.

Pharmacological Test Example II

Acute toxicity

The present substance was tested for acute toxicity by intravenously giving the substance at a dose of 19 mg protein/kg to ten 8-week-old ddY mice of each sex.

Consequently none of the animals died, while $LD_{50}$ was found to be at least 19.0 mg/kg. During the observation period, none of the animals exhibited poisoning symptoms which were apparently attributable to the present substance.

As described above, te present substance has cytotoxic activity or cytocidal activity on various cells, is low in toxicity and is therefore useful as an anti-tumor agent.

For use as an anti-tumor agent, the present substance is formulated into preparations of various forms which contain an effective amount of the substance.

The preparation is given to the patient or animal to be treated by a method suitable to the form thereof. Such preparations include oral preparations, for example, in the form of tablets, pellets, powder, solution, suspension, emulsion, granules and capsules, and non-oral preparations such as suppositories, injections (solutions, suspension, etc.) and the like. Tablets, pellets, solution, suspension, emulsion, granules and capsules are orally given. Injections are given singly or as admixed with usual supplimental solutions of glucose, amino acids or the like, intravenously. When used singly, injections are given intramuscularly, intracutaneously, subcutaneously or intraperitoneally. Suppositories are given to the rectum.

Such pharmaceutical preparations of various forms are formulated with use of carriers which are usually used and which include diluents or excipients such as fillers, extenders, binders, humectants, disintegrators, disintegration suppressants, surfactants (absorption promoters, adsorbents, etc.) and glazing agents. When required, coloring agents, preservatives, antiseptics, perfumes, flavoring agents, sweetening agents, etc., as well as other drugs, may be additionally used. Carriers for preparing tablets are a wide variety of those already known in the art and including excipients such as lactose, white sugar, sodium chloride, glucose, urea, starch, calcium carbonate, kaolin, crystalline cellulose and silicic acid; binders such as water, ethanol, propanol, syrup, glucose solution, starch solution, gelatin solution, carboxymethylcellulose, shellace, methylcellulose, potassium phosphate and polyvinylpyrrolidone; disintegrators such as dry starch, sodium alginate, agar powder, laminaria powder, sodium hydrogencarbonate, calcium carbonate, polyoxyethylene sorbitan fatty acid esters, sodium lauryl sulfate, glyceryl monostearate, starch and lactose; disintegration suppressants such as white sugar, stearin, cacao butter and hydrogenated oils; absorption promoters such as quaternary ammonium salt and sodium lauryl sulfate; humectants such as glycerin and starch; adsorbents such as starch, lactose, kaolin, bentonite and colloidal silicic acid; glazing agents such as purified talc, stearic acid salts, boric acid powder and polyethylene glycol; etc. When desired, tablets can be provided with usual coatings. Examples of such tablets are sugar-coated, gelatin-coated, enterically coated, film-coated, double-layer and multi-layer tablets. A wide variety of known carriers are usable for preparing pellets. Examples of such carriers include excipients such as glucose, lactose, starch, cacao butter, hardened vegetable oils, kaolin and talc; binders such as gum arabic powder, tragacanth powder, gelatin and ethanol; disintegrators such as laminaria and agar; etc. A wide variety of known carriers are usable for preparing suppositories. Examples of such carriers are polyethylene glycol, cacao butter, higher alcohols, esters of higher alcohols, gelatin, semi-synthetic glycerides, etc. The solutions, emulsions and suspensions prepared as injections are sterilized and are preferably isotonic with the blood. The diluents conventionally used in the art are all usable for preparing these solutions, emulsions and suspensions. Exemplary of such diluents are water, ethyl alcohol, propylene glycol, ethoxidized isostearyl alcohol, polyoxidized isostearyl alcohol, polyoxyethylene sorbitan fatty acid esters, etc. In this case, sodium chloride, glucose or glycerin may be incorporated into the pharmaceutical preparation in an amount sufficient to afford an isotonic solution. Also usable are usual auxiliary solubilizers, buffers, stabilizers, analgesics, etc. Injections can be prepared as dry preparations, such as freeze-dried powders made by known freeze-drying means, which can be made into liquids by addition of a suitable carrier before use.

The dosage of the pharmaceutical preparation (anti-tumor composition) differs according to the form of the preparation, tract of administration, symptoms, age and sex of the patient, etc. Usually the substance of the present invention is given at a dose of about 9.5 to about 95 µg/kg/day calculated as the amount of protein. It is desirable to give the preparation in divided doses per day.

The present invention will be described in greater detail with reference to the following reference example and examples, to which the invention is not limited.

### Reference Example 1

The following cells and anti-tumor substance inducing agents were used.

(1) Anti-tumor substance inducing agents

Sendai virus (HVJ, hemagglutinating virus of Japan), provided by K. Cantell, was inoculated into hen's egg on the 10th to 12th day of fertilization. Three days thereafter, the chorioallantoic fluid was collected and continuously ultracentrifuged (35000 r.p.m.) to purify the HVJ, which was suspended in PBS (-). The purified HVJ suspension was divided into suitable portions and frozen at -80°C for preservation before use.

The other anti-tumor substance inducing agents used were PHA (Difco Lab. Inc., U.S.A.), Con A (Wako Junyaku Co., Ltd.), LPS (E. coli 055: B5, Difco Lab. Inc., U.S.A.) and Corynebacterium parvum (Wellcome Reagent Ltd., Great Britain).

(2) Cells

For the production of the present substance, cells were prepared by incubating human cultured lymphoid cell lines (CCRF-CEM, DND-41, TALL-1, RPMI-8402 and CCRF-HSB-2 cells, all stated in Immunol. Commun., 9(8), pp. 731-734 (1980)) in a stationary state, using RPMI-1640 medium containing 10% FSC and an incubator maintained at 37°C and a carbon dioxide concentration of 5%.

The following procedure was also adopted for preparing the cells. Human lymphoid cells of each type ($1 \times 10^7$ cells) were subcutaneously transplanted in a newborn hamster (golden hamster grown by ourselves) within 24 hours after birth. A 0.1 ml quantity of rabbit anti-hamster thymus lymphocyte serum (ALS) was given to the hamster twice every week. The resulting tumor was removed 4 to 5 weeks after the transplant, washed with Eagle's MEM medium, finely divided by cutter and treated by a cell filter.

### Example 1

(1)     The cells of the human cultured lymphoid cell line of each type prepared in Reference Example 1-(2) were centrifuged and washed with Eagle's MEM medium, and a suspension of $2.5 \times 10^6$ cells/ml was prepared with use of RPMI-1640 medium containing 10% FCS.

A specified quantity of the anti-tumor substance inducing agent mentioned in Reference Example 1-(1) (i.e. 50 µg/ml of PHA, 10 µg/ml of Con A, 500 HA/ml of HVJ or 10 µg/ml of LPS) was added to the cell suspension and incubated at 37°C for 1 to 6 days with stirring. The culture supernatant obtained was centrifugally collected (10000 r.p.m.) and frozen at -20°C for preservation (crude solution).

Table 2 below shows the cytotoxic activity (potency, units/ml) of the resulting crude solution on L-cells. The potency was measured after the culture supernatant was centrifuged by filtration. Before the determination of potency, the HVJ-containing culture supernatant was subjected to UV treatment (for 30 minutes by 15-W UV lamp positioned 20 cm above the sample) to inactivate HVJ.

Table 2

| Cell | Anti-tumor substance inducing agent | | | |
| | PHA | Con A | HVJ | LPS |
| --- | --- | --- | --- | --- |
| DND-41 | 24 | 15 | 90 | 20 |
| TALL-1 | 60 | 15 | 63 | 15 |
| RPMI-8402 | 55 | 12 | 60 | 18 |
| CCRF-HSB-2 | 60 | 10 | 60 | 20 |
| CCRF-CEM | 60 | 10 | 130 | 20 |

(2)     In the same manner as above (1), 100 to 4000 HA/ml of HVJ was added to a suspension of CCRF-CEM, $5 \times 10^6$ cells/ml, in RPMI-1640 medium containing no serum, followed by incubation for 24 hours to obtain crude solutions.  Table 3 below shows the potencies (units/ml) thereof.

Table 3

| Crude solution No. | Amount of HVJ (HA/ml) | Potency of crude solution (units/ml) |
|---|---|---|
| 1 | 100 | 54 |
| 2 | 250 | 100 |
| 3 | 500 | 70 |
| 4 | 1000 | 90 |
| 5 | 2000 | 100 |
| 6 | 4000 | 100 |

(3)     PHA, 50 to 200 µg/ml, was added to a suspension of CCRF-CEM, $5 \times 10^6$ cells/ml, in RPMI-1640 medium containing 10% FCS, followed by incubation for 72 hours to obtain crude solutions (Nos.7 to 9) in the same manner as above (1).

Crude suspensions were prepared in the same manner as above with the exception of incubating the suspension for 48 hours after adding 50 µg/ml of PHA thereto, thereafter adding to the suspension 50 µg/ml of PHA (crude solution No.10), 50 µg/ml of C. parvum
(crude solution No.11) or 500 HA/ml of HVJ (crude solution
No.12), and incubating the resulting suspension further
for 4 days.  Table 4 below shows the potencies (units/ml)
of the solutions.

Table 4

| Crude solution No. | Anti-tumor substance inducing agent (µg or HA/ml) | Potency (units/ml) |
|---|---|---|
| 7 | PHA (50) | 69 |
| 8 | PHA (100) | 65 |
| 9 | PHA (200) | 80 |
| 10 | PHA (50) + PHA (50) | 80 |
| 11 | PHA (50) + C. Parvum (50) | 300 |
| 12 | PHA (50) + HVJ (500) | 150 |

(4) Isolation of the present substance

Ammonium sulfate (472 g) was added to and
dissolved in 1000 ml of the crude solution (54 units/ml)
prepared by causing HVJ (100 HA/ml) to act on CCRF-CEM
by the procedure (2).  The solution was thereafter
allowed to stand at 4°C overnight to precipitate an
active substance.  The precipitate was collected by
centrifuging (4°C) at 10000 r.p.m. for 30 minutes and
suspended in about 100 ml of 0.02M tris-hydrochloric
acid buffer/0.1M NaCl (pH 7.8).  This step achieved an activity collection ratio of 96% and a purifying degree of 18 times.

Subsequently 36.04 g of urea was dissolved in 100 ml of the precipitate suspension, and the solution was cold-centrifuged (4°C) at 10000 r.p.m. for 30 minutes to remove the insolubles. The supernatant (50 ml) was collected and subjected to gel filtration (column size 50 x 1000 mm) with use of Ultragel AcA54 equilibrated with 0.02M tris-hydrochloric acid/0.5M NaCl buffer. The fractions were checked for activity on L-cells to collect an active fraction. This achieved an activity collection ratio of 95% and a purifying degree of 47 times. The overall procedure achieved an activity collection ratio of 91.2% and a purifying degree of about 846 times.

Next, the active fraction was concentrated by an ultrafiltration-concentration device TCF-10 equipped with Amicon ultrafilter YM10. The concentrate was placed into a dialyzing tube and dialyzed at 4°C against 0.02M phosphoric acid buffer (pH 6.8) in 50 times the amount of the concentrate. The external liquid was replaced every 3 hours three times, and the dialyzate was thereafter allowed to stand in the same buffer overnight at 4°C. The active dialyzate fraction was passed through a column (size 26 x 400 mm) of hydroxyapatite gel equilibrated with 0.02M phosphoric acid buffer (pH 6.8) in advance.

The column was thoroughly washed with the same buffer under at a flow rate of 5.0 ml/hour and 3 ml/fraction, and the adsorbed substance was thereafter eluted by continuously elevating the concentration gradient with use of 500 ml of 0.02M phosphoric acid buffer (pH 6.8) and 500 ml of 0.5M phosphoric acid buffer (pH 6.8). With this method, no activity was found in the non-adsorption fractions, indicating complete adsorption of activity. The adsorbed substance was eluted at phosphoric acid buffer concentrations of between 0.15M to 0.3M. The active fractions were collected and concentrated by ultrafiltration at 4°C with use of Amicon ultrafiltration-concentration device TCF-10. This step achieved an activity collection ratio of 63% and a purifying degree of 6.3 times. The overall process achieved an activity collection ratio of 57.5% and a purifying degree of $5.33 \times 10^3$ times.

Subsequently the active concentrate obtained by the above step was placed into a dialyzing tube and dialyzed overnight at 4°C against 0.02M tris-hydrochloric acid (pH 7.8)/0.1M NaCl buffer in 50 times the amount of concetrate. The inside dialyzate was passed through Pharmacia mono Q column equilibrated with the same buffer, at 1 ml/min and 1 ml/fraction for the adsorption of active substance. After thoroughly washing the column with the same buffer, the adsorbed active fraction was eluted with 0.02M tris-hydrochloric acid (pH 7.8)/1.0M NaCl while increasing the concentration of NaCl continuously. The active fraction was concentrated by an ultrafiltration device equipped with Amicon ultrafilter YM10. This step achieved an activity collection ratio of 80% and an increased purifying degree of 5.1 times. The overall process achieved an activity collection ratio of 50% and a purifying degree of $2.72 \times 10^4$ times.

Through Toyo-pearl SW60 (column size 10 x 500 mm) equilibrated with 0.1M tris-hydrochloric acid (pH 7.8)/70% saturated ammonium sulfate, the concentrate was then passed at a flow rate of 1 ml/min and 2 ml/fraction to effect salting-out within the column, followed by elution with 0.1M tris-hydrochloric acid (pH 7.8) buffer at a continuously decreasing ammonium sulfate concentration. The active fractions were collected and concentrated by an ultrafiltration-concentration device equipped with Amicon ultrafilter YM10. This step achieved an activity collection ratio of 61% and a purifying degree of 3 times. The overall process achieved an activity collection ratio of 28.0% and a purifying degree of $8.15 \times 10^4$ times.

Next, the concentrate was subjected to gel filtration at a flow rate of 1 ml/min and 1 ml/fraction, using Toyo Soda G3000 SW column (7.5 x 600 mm) equilibrated with 1M sodium phosphate (pH 7.0)/0.2M NaCl/0.1% SDS buffer. This step achieved an activity collection ratio of 26% and an increased purifying degree of 2.9 times. The overall process achieved an activity collection ratio of 7.29% and a purifying degree of 2.35 x 10$^5$ times.

The above process gave a protein of this invention, which exhibits the foregoing physiological activity and physicochemical properties. The protein having such physiological activity has specific activity of 3.54 x 10$^6$ units/mg protein.

(5)     A protein of the invention was prepared in the same manner as in the above procedure (4) with the exception of using a crude solution (24 units/ml) obtained by causing PHA to act on DND-41 in the above procedure (1). The overall process achieved an activity collection ratio of 5.57%, a purifying degree of 4.92 x 10$^5$ times and specific activity of 3.49 x 10$^6$ units/mg protein.

(6)     A protein of the invention was prepared in the same manner as in the procedure (4) with the exception of using a crude solution (63 units/ml) obtained by causing HVJ to act on TALL-1 in the above (1). The overall process achieved an activity collection ratio of 5.52%, a purifying degree of $1.95 \times 10^5$ times and specific activity of $3.51 \times 10^6$ units/mg protein.

(7)     A protein of this invention was prepared in the same manner as in the procedure (4) with the exception of using a crude solution (12 units/ml) which was obtained by causing Con A to act on RPMI-8402 in the procedure (1). The overall process achieved an activity collection ratio of 7.45%, a purifying degree of $8.87 \times 10^5$ times and specific activity of $3.55 \times 10^6$ units/mg protein.

(8)     A protein of the invention was prepared in the same manner as in the procedure (4) with the exception of using a crude solution (10 units/ml) which was obtained in the above (1) by causing Con A to act on CCRF-HBS-2. The overall activity collection ratio was 6.4%, purifying degree of $9.52 \times 10^5$ times and specific activity of $3.52 \times 10^6$ units/mg protein.

(9)     In the same manner as in the procedure (4) and with use of the crude solutions as obtained by the procedures (1) to (3), proteins of this invention were prepared which had the same properties as above. The process was comparable to the foregoing in activity collection ratio, purifying degree and specific activity.

A preparation example is given below in which the substance of the invention was used.

## Preparation Example 1

Physiological NaCl aqueous solution (100 ml) containing $1 \times 10^9$ units of the present protein was sterilized by filtration, then divided into 2-ml portions and freeze-dried, giving an anti-tumor injection composition.

CLAIMS:

1. A protein derived and produced by causing an anti-tumor substance inducing agent to act on a human cultured lymphoid cell line, the protein having the following characteristics:

a) molecular weight: 56000 $\pm$ 3000,

b) isoelectric point: pH 4.9 $\pm$ 0.3,

c) being colorless and transparent when dissolved in 0.02M tris-hydrochloric acd buffer (pH 7.8) at a concentration of 3 mg protein/ml, the solution producing a precipitate when at least 70 V/V % of acetone or ethanol is added thereto,

d) being weakly acidic when in the form of an aqueous solution,

e) undergoing peptide bond and amino acid color reactions when subjected to burette reaction, Folin-Lowry reaction, and ninhydrin reaction after hydrolysis with hydrochloric acid,

f) having direct cytotoxic activity in vitro on cultured mouse L-cells, and

g) having anti-tumor activity on Meth A-sarcoma bearing mice in vivo.

2. A protein as defined in claim 1 wherein the anti-tumor substance inducing agent is selected from among viruses, endotoxin derived from gram-negative bacteria, lectin derived from vegetables and immuno-active substances.

3. A protein as defined in claim 2 wherein the anti-tumor substance inducing agent is selected from among lipopolysaccharides (LPS) derived from E. coli, Pseudomonas aeruginosa and Salmonella typhimrium, Concanavalia Ensiformis lectin (concanavalin A, Con A), soybean lectin (SBA), Phaseolus Vulgaris lectin (PHA), Sendai virus (HVJ), bacillus Calmette Guerin (BCG), Corynebacterium parvum, Propionibacterium acnes, Mycobacterium butyricum, Corynebacterium granulosum, Streptococcus pyrogenes, Plasmodium, Zymosan, Nocardia asteroides, Lysteria monocytogenes, glucan, cellwall skelton, dextran sulfate, muramyldipeptide, and krestin (product of Kureha Kogyo Co., Ltd.).

4. A protein as defined in claim 3 wherein the anti-tumor substance inducing agent is selected from among LPS, Con A, PHA, HVJ and Corynebacterium parvum.

5. A protein as defined in any one of claims 1 to 4 wherein the human cultured lymphoid cell line is selected from among CCRF-CEM, DND-41, TALL-1, RPMI-8402 and CCRF-HSB-2.

6. A protein as defined in claim 4 wherein the human cultured lymphoid cell line is CCRF-CEM.

7. A protein as defined in claim 1 which is obtained by causing HVJ to act on DND-41.

8. A protein as defined in claim 1 which is obtained by causing HVJ to act on CCRF-CEM.

9. A protein as defined in claim 1 which is obtained by causing HVJ and PHA to act on CCRF-CEM.

10. A protein as defined in claim 1 which is obtained by causing PHA and Corynebacterium parvum to act on CCRF-CEM.

11. A process for preparing a protein having anti-tumor activity characterized by causing an anti-tumor substance inducing agent to act on a human cultured lymphoid cell line and collecting a protein thereby derived and produced, the protein having the following characteristics:

a) molecular weight: 56000 ± 3000,

b) isoelectric point: pH 4.9 ± 0.3,

c) being colorless and transparent when dissolved in 0.02M tris-hydrochloric acd buffer (pH 7.8) at a concentration of 3 mg protein/ml, the solution producing a precipitate when at least 70 V/V % of acetone or ethanol is added thereto,

d) being weakly acidic when in the form of an aqueous solution,

e) undergoing peptide bond and amino acid color reactions when subjected to burette reaction, Folin-Lowry reaction, and ninhydrin reaction after hydrolysis with hydrochloric acid,

f) having direct cytotoxic activity <u>in vitro</u> on cultured mouse L-cells, and

g) having anti-tumor activity on Meth A-sarcoma bearing mice <u>in vivo</u>.

12. An anti-tumor composition comprising an effective amount of a protein derived and produced by causing an anti-tumor substance inducing agent to act on a human cultured lymphoid cell line, the protein having the following characteristics:

a) molecular weight: 56000 ± 3000,

b) isoelectric point: pH 4.9 ± 0.3,

c) being colorless and transparent when dissolved in 0.02M tris-hydrochloric acd buffer (pH 7.8) at a concentration of 3 mg protein/ml, the solution producing a precipitate when at least 70 V/V % of acetone or ethanol is added thereto,

d) being weakly acidic when in the form of an aqueous solution,

e) undergoing peptide bond and amino acid color reactions when subjected to burette reaction, Folin-Lowry reaction, and ninhydrin reaction after hydrolysis with hydrochloric acid,

f) having direct cytotoxic activity <u>in vitro</u> on cultured mouse L-cells, and

g) having anti-tumor activity on Meth A-sarcoma bearing mice <u>in vivo</u>.

# INTERNATIONAL SEARCH REPORT

0134247

International Application No. PCT/JP84/00019

## I. CLASSIFICATION OF SUBJECT MATTER (if several classification symbols apply, indicate all) 3

According to International Patent Classification (IPC) or to both National Classification and IPC

Int. Cl³  C07G 7/00, C12P 21/00, A61K 37/02
// (C12P 21/00, C12R 1/91)

## II. FIELDS SEARCHED

| Minimum Documentation Searched 4 | |
|---|---|
| Classification System | Classification Symbols |
| IPC | C07G 7/00, C12P 21/00, A61K 37/02 |

| Documentation Searched other than Minimum Documentation to the Extent that such Documents are Included in the Fields Searched 5 |
|---|
| |

## III. DOCUMENTS CONSIDERED TO BE RELEVANT 14

| Category* | Citation of Document, 16 with indication, where appropriate, of the relevant passages 17 | Relevant to Claim No. 18 |
|---|---|---|
| A | JP, A, 58-88322 (Hayashibara Seibutsu Kagaku Kenkyusho Kabushiki Kaisha, Mochita Pharmaceutical Co., Ltd.) 26 May 1983 (26. 05. 83) | 1 - 12 |
| A | JP, A, 58-146293 (Mochita Pharmaceutical Co., Ltd.) 31 August 1983 (31. 08. 83) | 1 - 11 |
| A | JP, A, 58-148826 (Mochita Pharmaceutical Co., Ltd. 5 September 1983 (05. 09. 83) | 1, 12 |
| A | Chemical Abstracts, Vol. 92, No. 21 26 May 1980 (26. 05. 80) (Columbus, Ohio. U.S.A.) Eardley, Diane D. et al, "Lymphotoxin production by subsets of T cells". See page 469, abstract No 178942 W, J. Immunol. 1980, 124(3)1199-202 (Eng) | 11 |

* Special categories of cited documents: 15

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search 2 | Date of Mailing of this International Search Report 2 |
|---|---|
| April 20, 1984  (20. 04. 84) | May 1, 1984  (01. 05. 84) |
| International Searching Authority 1 | Signature of Authorized Officer 20 |
| Japanese Patent Office | |

Form PCT/ISA/210 (second sheet) (October 1981)